# EUROPEAN PATENT SPECIFICATION

(11) **EP 3 838 325 B1**
(45) Date of publication and mention of the grant of the patent: **13.11.2024**
(21) Application number: 19216804.5
(22) Date of filing: 17.12.2019
(51) Int. Cl.: A61M 25/01, A61M 25/00, B33Y 80/00

(54) **ARTICULATING SHAFT FOR A STEERABLE CATHETER SYSTEM AND FABRICATION METHOD**
GELENKSCHAFT FÜR EIN LENKBARES KATHETERSYSTEM UND HERSTELLUNGSVERFAHREN
ARBRE ARTICULÉ POUR UN SYSTÈME DE CATHÉTER ORIENTABLE ET PROCÉDÉ DE FABRICATION

(43) Date of publication of application: 23.06.2021
(73) Proprietor: Creganna Unlimited Company, Ballybrit, Galway H91 VN2T (IE)
(72) Inventor: MULDOON, Damian, Galway, H62 KPO3 (IE); MCDERMOTT, Bernard, Claremorris, F12PD69 (IE)
(74) Representative: Grünecker Patent- und Rechtsanwälte PartG mbB

(56) References cited:
- EP-A1- 2 949 262
- WO-A1-2004/105849
- US-A- 4 911 148
- US-A- 6 012 494

## Description

The present invention relates to an articulating shaft for a steerable catheter system which may be usable with a minimally invasive procedure e. g. with an intravascular medical treatment system. The present invention also relates to a method of fabricating such a shaft.

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure in which an artificial valve or valve repair device is guided to the heart through the patient's vasculature. For example, a catheter is inserted into the patient's vasculature and directed to the inferior vena cava. The catheter is then urged through the inferior vena cava toward the heart by applying force longitudinally to the catheter. Upon entering the heart from the inferior vena cava, the catheter enters the right atrium. The distal end of the catheter may be deflected by one or more deflecting mechanisms, which can be achieved by a tension cable, or other mechanisms positioned inside the catheter. Precise control of the distal end of the catheter allows for more reliable and faster positioning of a medical device and/or implant and other improvements in the procedures. Apart from structural heart applications, the catheters are also used for minimally invasive procedures such as neurovascular, coronary, structural heart, peripheral vascular or endoscopic type procedures for gastrointestinal applications or other.

An intravascular delivered device needs to be placed precisely to ensure a correct positioning of the medical device, which is essential for its functionality, as the device may be difficult to reposition after the device is fully deployed from the delivery system. Additionally, catheters are required to have the ability to turn or rotate the distal end of the catheter with like-for-like movement of the proximal section or catheter handle. It is achieved through torque transfer along the length of the shaft. For example, single steer to traverse an anatomical challenge. At the same time, catheters are required to achieve movement of parts of the catheter independent of the rest of the catheter. The design of the catheter shaft is a significant factor in determining the formation of curves, angles of deflection and levels of steerability. The choice of material determines the level of pushability, torque, and flexibility and it can be manipulated along the length of the catheter through a variety of means to achieve the desired results.

A catheter shaft needs to be placed precisely to ensure a correct positioning of the medical device. Multiple lumens may be created within catheters for the passage of guidewires, catheters, fluids, and gases. The number of lumens depends on the material and cross-sectional area. Lumens can be shaped to meet user requirements. Reinforcement bars (or wires) and pull wires may be inserted in the lumens. A core lumen is usually provided for receiving the catheter with the medial device. Such a core lumen needs to be sealed hermetically in order to protect the medical device.

It is known to use single or multilumen shafts and braided, coiled or other layers arranged thereon for enhanced torqueability and deflection. Reinforcement bars and pull wires may be placed in situ during the braiding process. Lack of symmetry in the braid reinforcement layers due to the presence of varying thickness of wire height lead to suboptimal and often poor torque performance through the length of the catheter shaft. Additionally, problems may occur during fabrication related to the integration of reinforcement or pull wires in the lumens, moreover, a complex braider setup has to be provided for assembling the additional braided layers, thus increasing the fabrication costs.

EP2949262 describes articulating mechanisms, and flexible members and flexible segments that can form such articulating mechanisms. The mechanisms are useful, for example, for remote steering, guidance and/or manipulation of various instruments and tools at a targeted location.

US6012494 describes a flexible structure, a number n of notches is formed in a preferably circular-cylindrical block of material. The parallel and equidistant notches begin at two diametrically opposed jacket lines and are at right angles or an acute angle to the longitudinal axis of the block and are made so deep that for joining together (n+1) segments, a continuous, approximately rectangular residual cross-sectional region remains, in the form of a residual web. Symmetrically to the longitudinal axis, continuous, closed conduits are formed in the rectangular residual cross-sectional region. Moreover, aligned bores are located at diametrically opposed points, and in them pressure- and/or tension exerting components can be accommodated for bending the flexible structure.

WO2004105849 describes a hollow bendable tube with a specified diameter, comprising one or a plurality of slits axially formed from one side of the tip part thereof at specified intervals. The slits are formed perpendicular to the longitudinal direction of the bendable tube and extended toward the opposite side of the bendable tube, and a bending operation mechanism is installed in the hollow part of the bendable tube. The bendable tube can be manufactured by injection molding, and even if the radius of curvature thereof is small, the tube can be bent with a smaller bending force by a simple structure.

US4911148 describes an endoscope having a deflectable tip configured so as to have a small outer diameter of approximately 3,81 mm (0.15 inch) or less. The endoscope includes a flexible shaft subassembly comprised of a conduit having a deflectable end segment at its distal end which can be controlled by a manually operable mechanism on a handle subassembly.

There is still a need for an articulating shaft that has improved mechanical properties and allows the hermetic sealing of the core lumen, at the same time improving the fabrication process and reducing the costs of production.

This object is solved by the subject matter of the independent claims. Advantageous embodiments of the present invention are the subject matter of the dependent claims.

The present invention is based on the idea to fabricate an articulating shaft for a steerable catheter as one single piece, so that the central lumen for inserting the catheter is completely sealed along the entire length of the shaft. The central lumen may also be referred to as the "main lumen" or "core lumen". For allowing a steering of the shaft, at least one steering wire (also called "pull wire" or "actuating wire" in the following) can be inserted into a steering wire lumen that is formed by a plurality of aligned openings which are provided in wire support elements. These wire support elements are separated from each other by slots so that bending of the shaft is facilitated. These slots may also be referred to as "notches". The outer wall of the shaft contains such notches to allow the shaft to contract and bend on this side.

In particular, the present invention provides an articulating shaft for a steerable catheter system, the shaft comprising a tubular body with a longitudinal central axis, the tubular body having at least one sealed, i. e. radially closed lumen with a distal opening and a proximal opening, and a plurality of wire support elements for supporting at least one actuating wire. Each of the wire support elements comprise at least one feed-through opening for receiving the actuating wire, wherein two adjacent wire support elements are separated from each other in an axial direction by a slot, and wherein the body and the wire support elements are integrally formed from a single piece.

An advantage of this solution can be seen in the fact that a hermetic sealing of the core lumen can be achieved easily without additional covers or sheaths. Moreover, the assembly process is facilitated due to the one-piece design of the shaft.

According to an advantageous embodiment of the present invention, the slots extend by at least 45° in a radial direction around the body. Thus, a sufficiently large segment of the shaft has enough pliability to facilitate the steering process. For instance, each slot may be provided to extend around approximately half the circumference of the shaft. Each slot may have a gradually diminishing depth in a radial direction towards the slot's peripheral regions around the circumference of the shaft. This design allows for an optimized force distribution when bending the shaft.

In order to further enhance the flexibility of the shaft for a bending caused by the actuating wire, each wire support member may be formed to comprise at least one bracket, which is separated from the body by a void, and wherein the feed-through opening is arranged at the bracket. Advantageously, the bracket is shaped symmetric with respect to a mirror plane orthogonally intersecting the cross-section and the central longitudinal axis of the shaft. In particular, the feed-through opening advantageously is arranged in the middle of the bracket.

In order to allow the use of the shaft with a two-way steering system, i. e. with two pull wires pulling the shaft in two different, preferably opposing, directions, the shaft may comprise a first and a second row of wire support elements axially extending along the longitudinal axis and having first and second feed-through openings, respectively, the rows being arranged opposite to each other in a radial direction, wherein the first feed-through openings are aligned opposite to the second feed-through openings. Again, the wire support members may be constructed is brackets having a void between the feed-through opening and the body.

According to an advantageous example, the shaft has a circular radial outline. Such a design is compatible with existing steering systems and allows optimal force distribution.

In case of a two-way steering shaft, it is advantageous to arrange the closed main lumen concentrically within the outline of the shaft.

On the other hand, for an articulating shaft, which has to be bent only in one direction, it is advantageous to arrange the main lumen offset from the central axis, so that the closed lumen is arranged within the outline of the shaft with its central axis being distanced from the longitudinal central axis of the shaft. In this case, a larger part of the shaft's cross-section can be used for the wire support elements.

According to an advantageous example, the shaft is fabricated from a 3D printed biocompatible material. 3D printing has become an increasingly cost effective and accurate technology for producing medical material. 3D printing is an additive manufacturing (AM) process defined as a process of joining materials to make objects from 3D model data, usually layer upon layer, as opposed to subtractive manufacturing methodologies, such as traditional machining. 3D printing can deliver parts of very sophisticated and complex geometries with no need of post-processing, built from custom-made materials and composites with near-zero material waste, while being applicable to a diversity of materials, including smart materials such as shape memory polymers and other stimulus-responsive materials, or biocompatible materials. Therefore, 3D printing is a technology that allows designers and engineers to create unique products that can be manufactured at low volumes in a cost-effective way. In the field of medical engineering, 3D printing even allows to produce the shafts custom made and adapted to specific patients.

According to a further advantageous example, the shaft further comprises at least one reinforcement lumen for receiving a reinforcing wire. The reinforcing wires, which are assembled into these lumens, can be made from steel or Nitinol or other suitable materials with some elastic properties. The reinforcing wires can also be referred to as the "neutral axis support wires".

When the actuating wire is attached to the distal end of the assembly and a tensile load is applied then the wire shortens. This causes the notches to close, causing the assembly to bend in the direction of the notches. The neutral axis support wires, which are also attached at the distal end, bend to allow the assembly to bend. The neutral axis support wires also carry some articulation axial load. The wires keep the segments in alignment and position, and also keep tension on the assembly, preventing the segments from separating.

In order to facilitate the bending, the reinforcement lumen may be segmented by peripheral regions of the slots.

Furthermore, also other lumens, for instance for electric cables or the like may also be provided at the shaft according to the present invention. These lumens can be attached to the inner surface of the outer unsealed wall or they can be attached to the outer surface of the inner sealed wall.

The present invention further relates to a method for fabricating a shaft for a steerable catheter system, using 3D printing to achieve the features required to allow articulation while also maintaining a sealed lumen, the method comprising:
providing a tubular body with a longitudinal central axis, the tubular body having at least one closed lumen with a distal opening and a proximal opening,
fabricating a plurality of wire support elements for supporting at least one actuating wire, wherein each of the wire support elements comprise at least one feed-through opening for receiving the actuating wire, wherein two adjacent wire support elements are separated from each other in an axial direction by a slot, and wherein the body and the wire support elements are integrally formed from a single piece.

As mentioned above, a hermetic sealing of the core lumen can be achieved easily without additional covers or sheaths. Moreover, the assembly process is facilitated due to the one-piece design of the shaft.

A particularly high freedom of design with low cost can be achieved. if the shaft is fabricated by means of a 3D printing process, as mentioned above. However, the shaft may also be fabricated by means of an extrusion process or an injection molding process. These processes are advantageous for high-volume production at low cost.

According to an advantageous embodiment, the shaft is fabricated from a polymer material having a durometer in the range of 30 Shore A to 70 Shore A. It is particularly advantageous to manufacture the shaft from a polymer material having a durometer in the range of 35 Shore A to 40 Shore A. For instance, polyamide is a possible material. However, the shaft may also be fabricated from any other suitable material. A material with a relatively low hardness is desirable if low pulling forces and a soft surface are sought, a harder material is advantageous, if an enhanced stiffness is needed.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:
- **FIG. 1**: is a schematic perspective representation of a shaft according to a first example;
- **FIG. 2**: is a detail of Fig. 1;
- **FIG. 3**: is a front view of the shaft shown in Fig. 1;
- **FIG. 4**: is a first side view of the shaft shown in Fig. 1;
- **FIG. 5**: is a further side view of the shaft shown in Fig. 1;
- **FIG. 6**: is a first schematic sectional view indicated in Fig. 5;
- **FIG. 7**: is a further schematic sectional view indicated in Fig. 5;
- **FIG. 8**: is a further schematic sectional view indicated in Fig. 5;
- **FIG. 9**: is a schematic perspective representation of a shaft according to a second example.

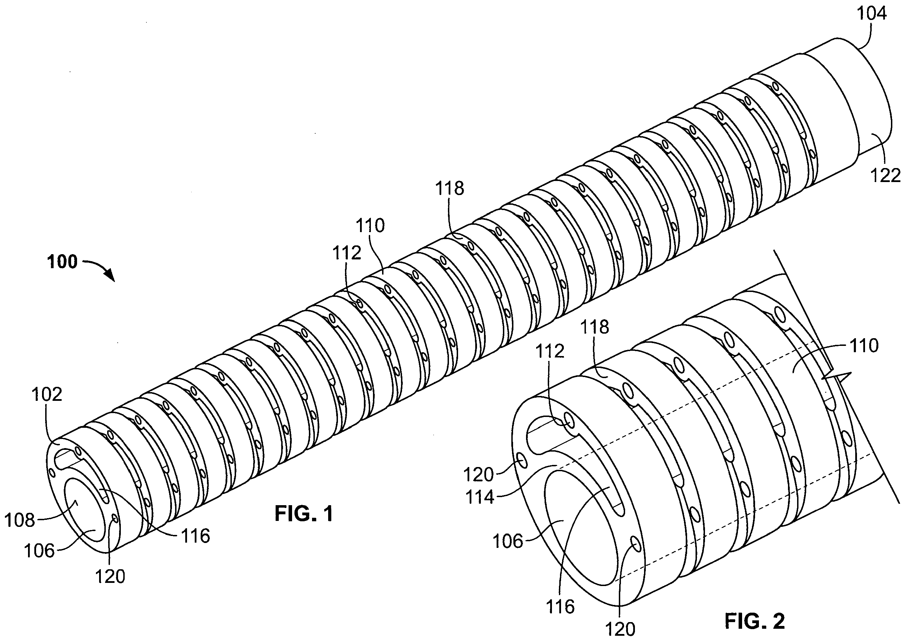

The present invention will now be explained in more detail with reference to the Figures and firstly referring to Fig. 1 and 2.

Fig. 1 shows a schematic perspective representation of an articulating shaft 100 according to a first example of the present invention, Fig. 2 is an enlarged view of the distal end 102 of the shaft 100. The shaft 100 has a distal end 102 with a distal opening and a proximal end 104 with a proximal opening. As used herein, the terms "proximal" and "distal" are to be taken as relative to a user using the disclosed delivery devices. "Proximal" is to be understood as relatively closer to the user and "distal" is to be understood as relatively farther away from the user.

A main lumen 106 extends along the complete length of the shaft 100. According to the present invention, the main lumen is delimited by an inner wall, which is closed along the length of the shaft 100. Thus, the main lumen can be sealed hermetically by connecting the distal and proximal ends 102, 104 to respective sealed fittings or connectors (not shown in the drawings).

According to the example shown in Fig. 1, the articulating shaft 100 comprises wire support elements 110 with feed-through openings 112. The feed-through openings are linearly arranged in the longitudinal direction of the shaft 100, so that a segmented lumen for the steering wire is formed. It should be noted that in the drawings the steering wires and other elements that are inserted into the various lumens are not shown. Each of the wire support elements 110 is formed as a bracket which is separated from a body 114 of the shaft 100 and by a void 116.

Slots 118 separate each of the brackets 110 from the adjacent one. These slots 118 facilitate the bending of the articulating shaft 100 if a pull wire is inserted into the row of feed-through openings 112 and exerts bending forces on the shaft 100. The presence of the voids 116 further enhances the flexibility and pliability of the shaft 100.

The shaft 100 according to the first example further comprises reinforcement lumens 120 for receiving reinforcement bars or wires (not shown in the Figures). These reinforcement wires may for instance be made from steel, Nitinol, or other suitable materials having some elastic properties. The reinforcement wires may also be referred to as the neutral axis support wires because they stabilize the segments formed by the slots 118 in their alignment and position. When a tensile load is applied, the actuating wire shortens and causes the notches 118 to close, thereby causing the assembly to bend in the direction of the notches 118. The neutral axis support wires arranged in the reinforcement lumens 120 also bend in order to allow the assembly to move. The reinforcement wires also carry some articulation axial load and keep tension on the assembly, thereby preventing the segments from separating.

In the shown embodiment two reinforcement lumens 120 are provided. It is clear for a person skilled in the art, however, that any other number of lumens may also be provided. Further, also additional lumens for electrical cables or fluid channels can be provided.

As shown in Fig. 1, at the proximal end 104 a fluidic connector 122 may be provided for a sealed connection to for instance a rigid part of a catheter or to a handle (not shown in the Figures). Of course, also at the distal end 102 a similar fluidic connector may be provided. Thereby, a complete sealing of the main lumen 106 can be achieved. If the shaft is intended to contain electric cables, the connector 122 may also comprise means for forming an electrical connection.

In the shown example, the shaft 100 has an essentially circular outer contour. However, any other cross-sectional outline may also be chosen, for instance an oval or polygonal contour. As shown here, also all openings forming a lumen have a circular cross-section. This is not necessarily the case; any other suitable cross-section, e. g. oval or polygonal, may also be used.

According to an advantageous aspect of the present invention, the articulating shaft 100 is formed as a 3D printed part, additively building up the part along the longitudinal axis of the shaft 100.

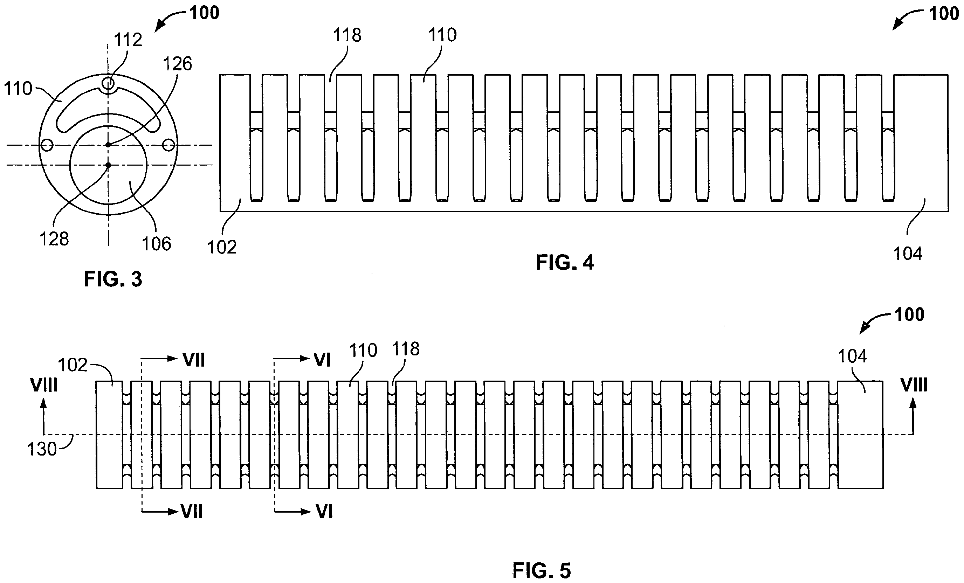

Further views of the articulating shaft according to the example shown in Figures 1 and 2 are depicted in Figures 3 to 8.

As may be derived from the frontal view of Fig. 3, the main lumen 106 has a center 128 which is off set with respect to a center 126 through which the longitudinal axis 130 of the shaft is passing. Thus, more room can be given to the bracket 110 comprising the feed-through opening 112 and the pliability of the shaft 100 is enhanced.

The first and second lumens 120 for the neutral axis support wires are arranged at the body 114 in a way that they are directly opposing each other along a diameter leading through the center 126 of the shaft 100.

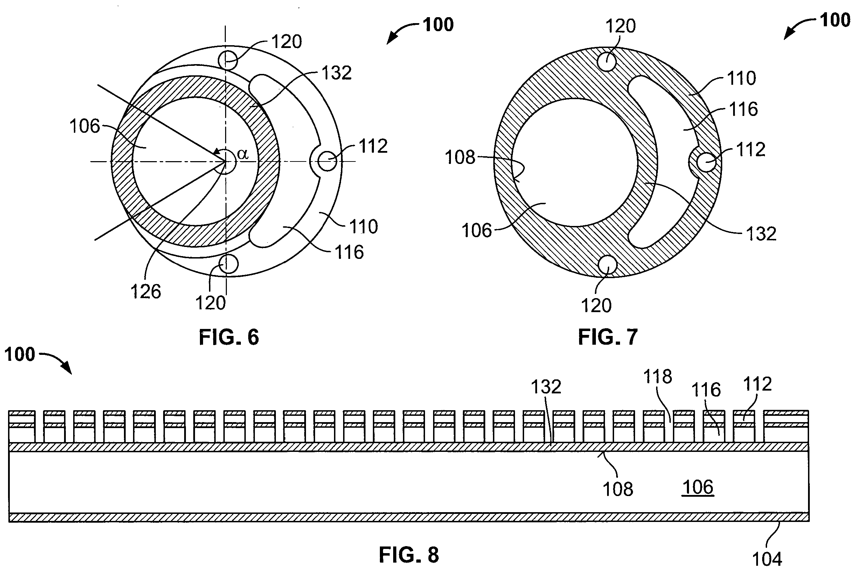

Figures 4 and 6 illustrate that the slots 118 which separate the plurality of segments formed by the wire support elements 110 from each other lead around the center 126 of the shaft 100 to cover a relatively large angle α, for instance 270°. Only the wall 132 forming the main lumen 106 remains closed continuously along the complete length of the shaft 100 for providing a sealed inner lumen 106.

When the shaft is bent in a direction toward the feed-through openings 112, the notches 118 close, but also the brackets 110 formed by the voids 116 can be deformed if necessary, so that the distance between the feed-through openings 112 and the wall 132 is reduced.

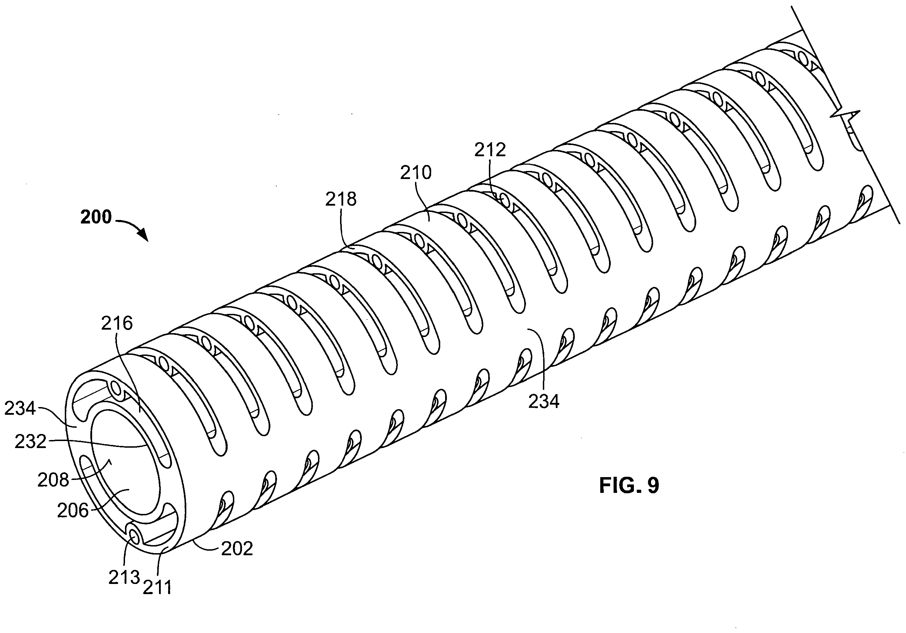

Fig. 9 illustrates a second example of an articulating shaft 200 according to the present invention. The shaft 200 has a distal end 202 with a distal opening and a proximal end (not visible in this Figure) with a proximal opening.

A main lumen 206 extends along the complete length of the shaft 200. According to the present invention, the main lumen 206 is delimited by an inner wall 208, which is closed along the length of the shaft 200. Thus, the main lumen 206 can be sealed hermetically by connecting the distal and proximal ends to respective sealed fittings or connectors (not shown in the drawings).

According to the example shown in Fig. 9, the articulating shaft 200 comprises first wire support elements 210 with first feed-through openings 212 and second wire support elements 211 with second feed-through openings 213. The feed-through openings 212, 213 are linearly arranged in the longitudinal direction of the shaft 200, so that two segmented lumens for two steering wires is formed. Thus, a two way steerable catheter can be provided using the shaft 200 shown in Fig. 9.

Again, it should be noted that in the drawings the steering wires and other elements that are inserted into the various lumens are not shown. Each of the wire support elements 210 is formed as a bracket which is separated from a body 214 of the shaft 200 and by a void 216.

Slots 218 separate each of the brackets 210 from the adjacent one. These slots 218 facilitate the bending of the articulating shaft 200 if a pull wire is inserted into the row of feed-through openings 212, 213 and exerts bending forces on the shaft 200. The presence of the voids 216 further enhances the flexibility and pliability of the shaft 200.

The design of the shaft 200 further differs from the design of the shaft 100 in that the cross-section is symmetrical, in other words, that the center of the main lumen 106 is coaxial with the central axis of the shaft 200.

When the shaft 200 is bent in a direction towards one of the rows of feed-through openings 212, the notches 218 close, but also the brackets 210 formed by the voids 216 can be deformed if necessary, so that the distance between the feed-through openings 212 and the wall 232 is reduced.

Further, each of the slots 218 extends along less than 180° of the outer circumference of the shaft 200, leaving two spines 234 for stabilizing the shaft 200. In the shown example, the spines 234 as such stabilize the neutral axis. However, it is clear for a person skilled in the art that one or more additional reinforcement lumens (similar to the ones shown with the first example of the shaft 100) can be embedded in the spines 234 for receiving a neutral axis support wire of bar. These reinforcement wires may for instance be made from steel, Nitinol, or other suitable materials having some elastic properties. The reinforcement wires may also be referred to as the neutral axis support wires because they stabilize the segments formed by the slots 218 in their alignment and position. When a tensile load is applied, the actuating wire on the respective side shortens and causes the notches 218 on the side of the actuated wire to close, thereby causing the assembly to bend in the direction of those notches 218. The neutral axis support wires arranged in the reinforcement lumens would also bend in order to allow the assembly to move. The reinforcement wires also carry some articulation axial load and keep tension on the assembly, thereby preventing the segments from separating.

Further, also additional lumens for electrical cables or fluid channels can be provided.

Although not visible in Fig. 9, at the proximal end of the shaft 200 a fluidic connector may be provided for a sealed connection to for instance a rigid part of a catheter or to a handle (not shown in the Figures). Of course, also at the distal end202 a similar fluidic connector may be provided. Thereby, a complete sealing of the main lumen 206 can be achieved. If the shaft is intended to contain electric cables, the connector may also comprise means for forming an electrical connection.

In the shown example, the shaft 200 has an essentially circular outer contour. However, any other cross-sectional outline may also be chosen, for instance an oval or polygonal contour. As shown here, also all openings forming a lumen have a circular cross-section. This is not necessarily the case; any other suitable cross-section, e. g. oval or polygonal, may also be used.

According to an advantageous aspect of the present invention, the articulating shaft 200 is formed as a 3D printed part, additively building up the part along the longitudinal axis of the shaft 200. However, it is clear that also other techniques of manufacturing the shaft 200 can be used. Preferably, 3D printing is used to achieve the features required to allow articulation while also maintaining a sealed lumen.

In summary, the present invention may provide an articulating shaft design based on 3D printed low durometer biocompatible material, containing a sealed main lumen. The 3D printed articulating design may contain one or more lumens for wires or cables and the sealed lumen may be placed concentric to the outer diameter or offset from the center axis. The outer wall contains notches to allow the shaft to contract and bend on this side.

Further the wire or cable lumens can be attached to the inner surface of the outer unsealed wall or they can be attached to the outer surface of the inner sealed wall. Additional wires may be assembled into these lumens. These can be steel or Nitinol or other suitable materials with some elastic properties. These will be referred to as the neutral axis support wires.

When these wires are attached to the distal end of the assembly and a tensile load applied then the wire (cable) shortens. This causes the notches to close, causing the assembly to bend in the direction of the notches. The neutral axis support wires bend to allow the assembly to bend. These wires also carry some articulation axial load. The wires keep the segments in alignment and position. The wires also keep tension on the assembly, preventing the segments from separating.

The notches can be configured in a multitude of ways, with single way and two way steering possible.

### REFERENCE NUMERALS

| **Reference Numeral** | **Description** |
|---|---|
| 100, 200 | Articulating shaft |
| 102, 202 | Distal end |
| 104, 204 | Proximal end |
| 106, 206 | Main lumen |
| 108, 208 | Inner wall of main lumen |
| 110, 210 | Wire support element |
| 211 | Second wire support element |
| 112, 212 | Feed-through opening |
| 213 | Second feed-through opening |
| 114, 214 | Body |
| 116, 216 | Void |
| 118, 218 | Slot |
| 120 | Reinforcement lumen |
| 122 | Connector |
| 126 | Center of shaft |
| 128 | Center of main lumen |
| 130 | Longitudinal axis |
| 132, 232 | Wall |
| 234 | Spine |

## Claims

1. Articulating shaft for a steerable catheter system, the shaft (100, 200) comprising:
a tubular body (114, 214) with a longitudinal central axis (130), the tubular body (114, 214) having at least one sealed lumen (106, 206) with a distal opening and a proximal opening, and
a plurality of wire support elements (110, 210) for supporting at least one actuating wire,
wherein each of the wire support elements (110, 210) comprise at least one feed-through opening (112, 212) for receiving the actuating wire, wherein two adjacent wire support elements (110, 210) are separated from each other in an axial direction by a slot (118, 218), and wherein the body (114, 214) and the wire support elements (110, 210) are integrally formed from a single piece,
wherein each wire support element (110, 210) comprises at least one bracket, which is separated from the body (114, 214) by a void (116, 216), and wherein the feed-through opening (112, 212) is arranged at the bracket.

2. Shaft according to claim 1, wherein the slots (118, 218) extend by at least 45° in a radial direction around the body (114, 214).

3. Shaft according to one of the preceding claims, wherein the shaft (200) comprises a first and a second row of wire support elements (210, 211) axially extending along the longitudinal axis and having first and second feed-through openings (212, 213), respectively, the rows being arranged opposite to each other in a radial direction, wherein the first feed-through openings (212) are aligned opposite to the second feed-through openings (213).

4. Shaft according to one of the preceding claims, wherein the shaft (100, 200) has a circular radial outline.

5. Shaft according to claim 4, wherein the sealed lumen (206) is arranged concentrically within the outline of the shaft (200).

6. Shaft according to claim 4, wherein the sealed lumen (106) is arranged within the outline of the shaft (100) with its central axis (128) being distanced from the longitudinal central axis (130) of the shaft (100).

7. Shaft according to one of the preceding claims, wherein the shaft (100, 200) is fabricated from a 3D printed biocompatible material.

8. Shaft according to one of the preceding claims, wherein the shaft (100, 200) further comprises at least one reinforcement lumen (120) for receiving a reinforcing wire.

9. Shaft according to claim 8, wherein the reinforcement lumen (120) is segmented by peripheral regions of the slots.

10. Method for fabricating a shaft (100, 200) for a steerable catheter system, the method comprising:
providing a tubular body (114, 214) with a longitudinal central axis (130), the tubular body (114, 214) having at least one sealed lumen (106, 206) with a distal opening and a proximal opening,
fabricating a plurality of wire support elements (110, 210) for supporting at least one actuating wire, wherein each of the wire support elements (110, 210) comprise at least one feed-through opening (112, 212) for receiving the actuating wire, wherein two adjacent wire support elements (110, 210) are separated from each other in an axial direction by a slot (118, 218), and wherein the body (114, 214) and the wire support elements (110, 210) are integrally formed from a single piece,
wherein each wire support element (110, 210) comprises at least one bracket, which is separated from the body (114, 214) by a void (116, 216), and wherein the feed-through opening (112, 212) is arranged at the bracket.

11. Method according to claim 10, wherein the shaft (100, 200) is fabricated by means of a 3D printing process.

12. Method according to claim 10 or 11, wherein the shaft is (100, 200) fabricated by means of an extrusion process.

13. Method according to one of the claims 10 to 12, wherein the shaft (100, 200) is 3D printed from a material having a durometer in the range of 30 Shore A to 70 Shore A.

14. Method according to claim 13, wherein the shaft (100, 200) is 3D printed from a polymer material having a durometer in the range of 35 Shore A to 40 Shore A.

## Patentansprüche

1. Gelenkiger Schaft für ein lenkbares Kathetersystem, wobei der Schaft (100, 200) umfasst:
einen röhrenförmigen Körper (114, 214) mit einer Längsmittelachse (130), wobei der röhrenförmige Körper (114, 214) mindestens ein abgedichtetes Lumen (106, 206) mit einer distalen Öffnung und einer proximalen Öffnung aufweist, und
eine Vielzahl von Drahstützelementen (110, 210) zum Unterstützen mindestens eines Betätigungsdrahtes,
wobei jedes der Drahtstützelemente (110, 210) mindestens eine Durchführungsöffnung (112, 212) zum Aufnehmen des Betätigungsdrahtes aufweist, wobei zwei benachbarte Drahtstützelemente (110, 210) in einer axialen Richtung durch einen Schlitz (118, 218) voneinander getrennt sind, und wobei der Körper (114, 214) und die Drahtstützelemente (110, 210) einstückig aus einem einzigen Stück gebildet sind,
wobei jedes Drahtstützelement (110, 210) mindestens einen Bügel umfasst, der durch einen Hohlraum (116, 216) vom Körper (114, 214) getrennt ist, und wobei die Durchführungsöffnung (112, 212) am Bügel angeordnet ist.

2. Schaft nach Anspruch 1, wobei sich die Schlitze (118, 218) um mindestens 45° in radialer Richtung um den Körper (114, 214) erstrecken.

3. Schaft nach einem der vorhergehenden Ansprüche, wobei der Schaft (200) eine erste und eine zweite Reihe von Drahtstützelementen (210, 211) aufweist, die sich axial entlang der Längsachse erstrecken und erste bzw. zweite Durchführungsöffnungen (212, 213) aufweisen, wobei die Reihen in radialer Richtung einander gegenüberliegend angeordnet sind, wobei die ersten Durchführungsöffnungen (212) gegenüber den zweiten Durchführungsöffnungen (213) ausgerichtet sind.

4. Schaft nach einem der vorhergehenden Ansprüche, wobei der Schaft (100, 200) einen kreisförmigen radialen Umriss aufweist.

5. Schaft nach Anspruch 4, wobei das abgedichtete Lumen (206) konzentrisch innerhalb des Umrisses des Schafts (200) angeordnet ist.

6. Schaft nach Anspruch 4, wobei das abgedichtete Lumen (106) innerhalb des Umrisses des Schafts (100) angeordnet ist und seine Mittelachse (128) von der Längsmittelachse (130) des Schafts (100) beabstandet ist.

7. Schaft nach einem der vorhergehenden Ansprüche, wobei der Schaft (100, 200) aus einem 3D-gedruckten biokompatiblen Material hergestellt ist.

8. Schaft nach einem der vorhergehenden Ansprüche, wobei der Schaft (100, 200) ferner mindestens ein Verstärkungslumen (120) zur Aufnahme eines Verstärkungsdrahtes aufweist.

9. Schaft nach Anspruch 8, wobei das Verstärkungslumen (120) durch Randbereiche der Schlitze segmentiert ist.

10. Verfahren zur Herstellung eines Schaftes (100, 200) für ein lenkbares Kathetersystem, wobei das Verfahren umfasst:
Bereitstellen eines röhrenförmigen Körpers (114, 214) mit einer Längsmittelachse (130), wobei der röhrenförmige Körper (114, 214) mindestens ein abgedichtetes Lumen (106, 206) mit einer distalen Öffnung und einer proximalen Öffnung aufweist,
Herstellen einer Vielzahl von Drahtstützelementen (110, 210) zum Unterstützen mindestens eines Betätigungsdrahtes, wobei jedes der Drahtstützelemente (110, 210) mindestens eine Durchführungsöffnung (112, 212) zum Aufnehmen des Betätigungsdrahtes aufweist, wobei zwei benachbarte Drahtstützelemente (110, 210) in einer axialen Richtung durch einen Schlitz (118, 218) voneinander getrennt sind, und wobei der Körper (114, 214) und die Drahtstützelemente (110, 210) einstückig aus einem einzigen Stück gebildet sind,
wobei jedes Drahtstützelement (110, 210) mindestens einen Bügel umfasst, der durch einen Hohlraum (116, 216) vom Körper (114, 214) getrennt ist, und wobei die Durchführungsöffnung (112, 212) am Bügel angeordnet ist.

11. Verfahren nach Anspruch 10, wobei der Schaft (100, 200) mittels eines 3D-Druckverfahrens hergestellt wird.

12. Verfahren nach Anspruch 10 oder 11, wobei der Schaft (100, 200) mittels eines Extrusionsverfahrens hergestellt wird.

13. Verfahren nach einem der Ansprüche 10 bis 12, wobei der Schaft (100, 200) aus einem Material mit einer Härte im Bereich von 30 Shore A bis 70 Shore A 3D-gedruckt wird.

14. Verfahren nach Anspruch 13, wobei der Schaft (100, 200) aus einem Polymermaterial mit einer Härte im Bereich von 35 Shore A bis 40 Shore A 3D-gedruckt wird.

## Revendications

1. Tige d'articulation pour un système de cathéter orientable, la tige (100, 200) comprenant :
un corps tubulaire (114, 214) ayant un axe longitudinal central (130), le corps tubulaire (114, 214) ayant au moins une lumière scellée (106, 206) avec une ouverture distale et une ouverture proximale, et
une pluralité d'éléments de support de fil (110, 210) pour supporter au moins un fil d'actionnement,
dans laquelle chacun des éléments de support de fil (110, 210) comprend au moins une ouverture à alimentation traversante (112, 212) pour recevoir le fil d'actionnement, dans laquelle deux éléments de support de fil (110, 210) adjacents sont séparés l'un de l'autre dans un sens axial par une fente (118, 218), et dans laquelle le corps (114, 214) et les éléments de support de fil (110, 210) sont intégralement formés d'une pièce unique,
dans laquelle chaque élément de support de fil (110, 210) comprend au moins un étrier, qui est séparé du corps (114, 214) par un vide (116, 216), et dans laquelle l'ouverture à alimentation traversante (112, 212) est agencée au niveau de l'étrier.

2. Tige selon la revendication 1, dans laquelle les fentes (118, 218) s'étendent d'au moins 45° dans un sens radial autour du corps (114, 214).

3. Tige selon l'une des revendications précédentes, dans laquelle la tige (200) comprend une première et une seconde rangée d'éléments de support de fil (210, 211) s'étendant axialement le long de l'axe longitudinal et ayant de premières et secondes ouvertures à alimentation traversante (212, 213), respectivement, les rangées étant agencées opposées l'une à l'autre dans un sens radial, dans laquelle les premières ouvertures à alimentation traversante (212) sont alignées opposées aux secondes ouvertures à alimentation traversante (213).

4. Tige selon l'une des revendications précédentes, dans laquelle la tige (100, 200) présente un contour radial circulaire.

5. Tige selon la revendication 4, dans laquelle la lumière scellée (206) est agencée de manière concentrique à l'intérieur du contour de la tige (200).

6. Tige selon la revendication 4, dans laquelle la lumière scellée (106) est agencée à l'intérieur du contour de la tige (100) avec son axe central (128) éloigné de l'axe longitudinal central (130) de la tige (100).

7. Tige selon l'une des revendications précédentes, dans laquelle la tige (100, 200) est fabriquée à partir d'un matériau biocompatible imprimé en 3D.

8. Tige selon l'une des revendications précédentes, dans laquelle la tige (100, 200) comprend en outre au moins une lumière de renfort (120) pour recevoir un fil de renfort.

9. Tige selon la revendication 8, dans laquelle la lumière de renfort (120) est segmentée par des régions périphériques des fentes.

10. Procédé de fabrication d'une tige (100, 200) pour un système de cathéter orientable, le procédé comprenant les étapes consistant à :
fournir un corps tubulaire (114, 214) avec un axe longitudinal central (130), le corps tubulaire (114, 214) ayant au moins une lumière scellée (106, 206) avec une ouverture distale et une ouverture proximale,
fabriquer une pluralité d'éléments de support de fil (110, 210) pour supporter au moins un fil d'actionnement, dans lequel chacun des éléments de support de fil (110, 210) comprend au moins une ouverture à alimentation traversante (112, 212) pour recevoir le fil d'actionnement, dans lequel deux éléments de support de fil (110, 210) adjacents sont séparés l'un de l'autre dans un sens axial par une fente (118, 218), et dans lequel le corps (114, 214) et les éléments de support de fil (110, 210) sont intégralement formés d'une pièce unique,
dans lequel chaque élément de support de fil (110, 210) comprend au moins un étrier, qui est séparé du corps (114, 214) par un vide (116, 216), et dans lequel l'ouverture à alimentation traversante (112, 212) est agencée au niveau de l'étrier.

11. Procédé selon la revendication 10, dans lequel la tige (100, 200) est fabriquée à l'aide d'un procédé d'impression en 3D.

12. Procédé selon la revendication 10 ou 11, dans lequel la tige (100, 200) est fabriquée à l'aide d'un procédé d'extrusion.

13. Procédé selon l'une des revendications 10 à 12, dans lequel la tige (100, 200) est imprimée en 3D à partir d'un matériau ayant une dureté au duromètre située dans la plage de 30 Shore A à 70 Shore A.

14. Procédé selon la revendication 13, dans lequel la tige (100, 200) est imprimée en 3D à partir d'un matériau polymère ayant une dureté au duromètre située dans la plage de 35 Shore A à 40 Shore A.
